# EUROPEAN PATENT APPLICATION

(11) **EP 4 821 441 A1**
(43) Date of publication of application: **14.10.2026**
(21) Application number: 25170009.2
(22) Date of filing: 11.04.2025
(51) Int. Cl.: A61B 6/03, A61B 6/00, G06T 7/00, G06T 7/70, G06T 7/80

(54) **PATIENT MONITORING DURING A SCAN**

(71) Applicant: Koninklijke Philips N.V., 5656 AE Eindhoven (NL)
(72) Inventor: SENEGAS, Julien Thomas, Eindhoven (NL); FRERKING, Lena Christina, Eindhoven (NL); BYSTROV, Daniel, Eindhoven (NL)
(74) Representative: Philips Intellectual Property & Standards

(57) **Abstract**

An imaging system captures images of a patient during a medical scan using a scanner having a scanning system and a patient support that is configured to move relative to the scanning system. A set of one or more optical cameras is mounted in a fixed position relative to the scanning system and directed towards the patient support for capturing images. A position input identifying a patient position is received, and virtual views are created by image post-processing. The post-processing involves adapting an orientation and/or focal depth for the virtual view generation in dependence on the patient position. A virtual view image sequence is then generated for display.

## Description

### FIELD OF THE INVENTION

The invention relates to monitoring a patient during a medical scan, in particular to detect movement.

### BACKGROUND OF THE INVENTION

In many medical imaging procedures, it is important to monitor a patient during the imaging procedure, for instance, to monitor movement of the patient during the procedure or to monitor the well-being of the patient during the imaging procedure. For example, monitoring images, such as video images provided by cameras, such as wide field of view cameras, are used for the monitoring.

By way of example, it is desirable to detect breathing-related movements in order to prevent and correct for motion artifacts, ensure optimal image quality, and support respiratory triggered scans. Indeed, undesired patient motion is one of the main reasons for image quality issues and safety events (like finger pinching).

Moreover, for these monitoring applications it is particularly important to monitor a predetermined region of interest of the patient. For instance, if breathing motions should be monitored, the chest of the patient has to be monitored, or, if a well-being of the patient should be monitored, the face of the patient has to be monitored. Since during certain medical imaging procedures, like CT imaging procedures or MR imaging procedures, the patient may be moved through the imaging device, for monitoring the region of interest of the patient, this region of the patient has to be tracked in the provided monitoring images.

It is difficult to provide images to enable the patient to be watched during the entire examination for all the different patient support positions, namely outside (front and rear) and inside the scanner bore. Typically, a dedicated camera and monitor is used for this purpose, but this requires manual camera adjustments thus interrupting a normal user workflow.

For monitoring patients inside a tight CT- or MR-bore, in-bore cameras have been proposed. They offer the advantage to provide an unobstructed view to the patient also when the patient is inside the bore. However, they also usually have a very narrow field-of-view (FOV) due to the bore limitations. Fisheye lenses, as typically used in surveillance systems, are well suited to cover a wide FOV. Fisheye images and videos are distorted and therefore a spatial orientation with fisheye lenses and recognizing anatomical structures is difficult for human observers as well as for AI systems, which are normally trained on less distorted perspective images.

Furthermore, the motion of the patient support appears highly curved in raw fisheye videos. This not only creates an unnatural visual effect, but it also makes it more difficult to visually interpret and predict the motion of the patient support.

One possible solution is to have an in-bore camera with a smaller FOV but equipped with a motor. The cost of such an approach would be higher and there may be interference with the magnetic and electrical fields especially for MR systems. This approach would also require more installation space behind the covers for the motor itself and for the additional cables, which makes the integration more difficult or not possible ultimately.

EP 4295744 discloses an imaging system for capturing images of a patient during a medical scan in which image post-processing is performed to correct distortions within the captured images resulting from the width of the field of view of the camera or cameras and from perspective distortion. At least one region of interest of the patient is tracked during patient support displacement and a respective distortion-corrected image sequence is generated of the or each region of interest. This imaging system thus creates a distortion-corrected view of a region of interest of a patient as they move with a patient support.

This approach addresses the problem that when the patient is moved through the medical imaging device, the shape and location of the region of interest will change in the monitoring image during the imaging procedure, due to the change of perspective of the monitoring camera with respect to the region of interest. This problem makes purely image based tracking of a region of interest to be monitored more difficult.

However, with the solution of EP 4295744 the image of the region of interest remains a portion of a (distortion-corrected) view from the fixed camera position, so with the same view angle. Thus, there is no flexibility to view images from an ideal viewpoint for the analysis of the region of interest. Also, the cropping of the image to select the region of interest is not likely to be perfect so it may vary slightly from frame to frame, and the target structure may thus move slightly between frames. This will be seen as jitter.

### SUMMARY OF THE INVENTION

The invention is defined by the claims.

According to examples in accordance with an aspect of the invention, there is provided an imaging system for capturing images of a patient during a medical scan using a scanner having a scanning system and a patient support that is configured to move relative to the scanning system, the imaging system comprising:
a set of one or more optical cameras for mounting in a fixed position relative to the scanning system directed towards the patient support for capturing images; and
a processor, characterized in that the processor is configured to:
   receive a position input identifying a patient support position or providing patient position information relative to the imaging system;
   perform image post-processing of the captured images to create virtual views, wherein the image post-processing comprises selecting (i) a position and orientation and/or (ii) a focal depth, for the virtual view generation in dependence on the position input;
   generate at least one virtual view image sequence; and
   output the virtual view image sequence for display.

This imaging system creates views of a patient as they move with a patient support. The position and orientation of each real camera relative to the scanner coordinate system is known, to enable the post-processing.

Depending on the position of the patient (known either based on the patient support position or the monitored actual position of the patient), virtual view settings are adapted (such as the virtual camera position, orientation or focal depth) dynamically so as to provide an optimal view. For example, the yaw angle may be controlled in dependence on the patient support position. Also, the focal length may be increased when a region of interest is further away from the camera.

The patient support position may be tracked as it is a known parameter of the imaging system. Alternatively, the position of the patient may be tracked for example by an external sensor, such as a 3D camera used for patient positioning or a CT image such as a survey image. It is known to use a 3D camera for example to know whether the patient is head first or feet first, with arms up or down, and / or where the individual body parts are located.

The position input (identifying the patient support position or the actual patient position) is in this way used as an input to define the virtual view parameters, for example including focal length of a virtual camera, and the position and the viewing angle of the virtual camera (such as the pitch, roll and yaw angles). These settings may all be adjusted in real time to perform a tracking mode.

This approach makes sure that the output of the processing is relevant to the scanning workflow and for the operator. It provides a logical link between the display output, namely the image sequence of the patient (such as the face of the patient) while the patient support is moving, and the scanning process, in particular the movement of the patient.

If using the patient support position as in input, instead of performing all analysis based on the images themselves, jitter or other incoherence between consecutive frames can be avoided.

The processor is for example configured to correct distortions within the captured images resulting at least from the width of the field of view of the camera or cameras. The imaging system thus creates distortion-corrected virtual views of a patient. The virtual camera views are then distortion-corrected views.

The processor may be configured to correct distortions by performing a pseudo-cylindrical mapping comprising mapping from each point in space onto a cylinder surface orthogonally towards the cylinder axis. This provides a computationally simple way to perform distortion correction.

The processor is for example configured to create virtual views of a tracked region of interest of the patient. The camera or cameras are close to the patient and they generate a distorted image to enable a wide field of view so that a region of interest can be tracked. The imaging system may be considered to digitally simulate the effect obtained by a motorized camera, making use of the large FOV. A virtual but natural traveling effect may for example be created of a region of interest, which appears as if the observer would turn the head to follow the moving object during patient support movement, such that the region of interest remains in the center of the image.

By tracking a region of interest, the part of the image intended to be viewed may be located at a static location within the output images. This means the region of interest can be inspected easily in the virtual image sequence. The virtual image sequence of the or each region of interest is for example a video stream of images that contain the tracked region of interest cropped out of the distortion-corrected images.

In addition to cropping the ROI within a fixed reconstructed image (as proposed in EP 4295744), a new different image is created corresponding to a virtual camera with adapted orientation and position and/or focal depth.

The processor is for example configured to perform the tracking using the position input.

The region or regions of interest for example comprise one or more of:
the face;
the hands;
the thorax, including the diaphragm region;
a body area to be scanned; and
a body area where there is an intravenous injection.

The processor is for example configured to adapt a virtual view orientation of the camera or cameras by adjusting one or more of a yaw angle, roll angle, pitch angle, camera position and focal length.

At least one camera of the set for example comprises a fisheye lens with a field of view greater than 120 degrees, for example greater than 150 degrees. Thus, the patient support (and the patient on the patient support) can be imaged by a small set of cameras, even with only a single camera.

The processor may be configured to perform a calibration process which involves imaging one or more calibration images. This calibration process enables the required distortion correction function to be derived. A communication system is for example provided for transmitting the virtual view image sequence to a screen or to a remote system.

The invention also provides a medical scanner comprising:
a scanning system;
a patient support extending through the scanning system;
a drive system for driving the patient support through the scanning system; and
the imaging system described above.

The medical scanner for example comprises a CT scanner, a PET scanner or an MRI scanner.

The set of cameras of the imaging system may comprise:
a first camera mounted above the patient support at one side of the scanning system, facing towards the patient support; and optionally
a second camera mounted above the patient support at an opposite side of the scanning system, facing towards the patient support.

The set of cameras may have only the first camera. The camera is for example at an edge of an opening through the scanning system, in particular an edge at an upper side of the scanning system. The camera faces at least partially downwardly. It may face vertically downwardly or it may be inclined to the vertical, for example giving a side view of the patient from above. It is desirable that the camera is looking orthogonally tow the patient support, i.e. the optical axis of the camera is perpendicular to the direction of movement of the patient support. This is not required, but facilitates the distortion correction.

There may be multiple cameras at the same side of the scanning system, for example at different angular positions around the scanning system.

When a second camera is mounted above the patient support at an opposite side of the scanning system, it may also be at an edge of the opening through the scanning system in particular an upper side of the scanning system. The use of two cameras, one at the front and one at the back, enables an increased combined field of view (or equivalently enables a smaller field of view to be used per camera). The use of two (or more) cameras also enables an occlusion-free view to the patient, whatever the patient support position is, as either the front or the rear camera will have a direct line of sight to the patient, especially to the face.

The invention also provides an imaging processing method for processing images of a patient during a medical scan using a scanner having a scanning system, a patient support and a set of one or more cameras mounted in a fixed position relative to the scanning system directed towards the patient support, characterized in that the method comprises:
receiving a position input identifying a patient support position or providing patient position information relative to the imaging system;
performing image post-processing of images captured by the set of one or more cameras to create virtual views, wherein the image post-processing comprises adapting (i) a position and orientation and/or (ii) a focal depth for the virtual view generation in dependence on the position input;
generating a virtual view image sequence; and
outputting the virtual view image sequence for display.

The method may comprise correcting distortions within the captured images resulting at least from the width of the field of view of the camera or cameras and creating virtual views of a tracked region of interest of the patient. The tracking is preferably performed using the position input.

The invention also provides a computer program comprising computer program code which is configured, when the program is run on the processor of the imaging system described above, to implement the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows schematically an example of a medical scanner;
Fig. 2 shows an example of three images of a distortion-corrected image sequence;
Fig. 3 shows a side view of a CT scanner with a single camera located at one side of a gantry;
Fig. 4 shows a side view of a CT scanner with two cameras; a first camera located at one side of the gantry and a second camera located at an opposite side of the gantry;
Fig. 5 shows two cameras located within the gantry bore with lateral positions;
Fig. 6 shows a pattern for use in a calibration stage;
Fig. 7 shows camera angle parameters;
Fig. 8 shows an image processing method implemented by the processor of the scanner of Figure 1;
Fig. 9 shows a conventional cylindrical projection;
Fig. 10 shows a pseudo-cylindrical projection;
Fig. 11 shows values used in the pseudo-cylindrical projection; and
Fig. 12 shows an example of an original camera image and virtual camera images derived from the original camera image.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an imaging system that captures images of a patient during a medical scan using a scanner having a scanning system and a patient support that is configured to move relative to the scanning system. A set of one or more optical cameras is mounted in a fixed position relative to the scanning system and directed towards the patient support for capturing images. A position input identifying a patient position is received, and virtual views are created by image post-processing. The post-processing involves adapting an orientation and/or focal depth for the virtual view generation in dependence on the patient position. A virtual view image sequence is then generated for display.

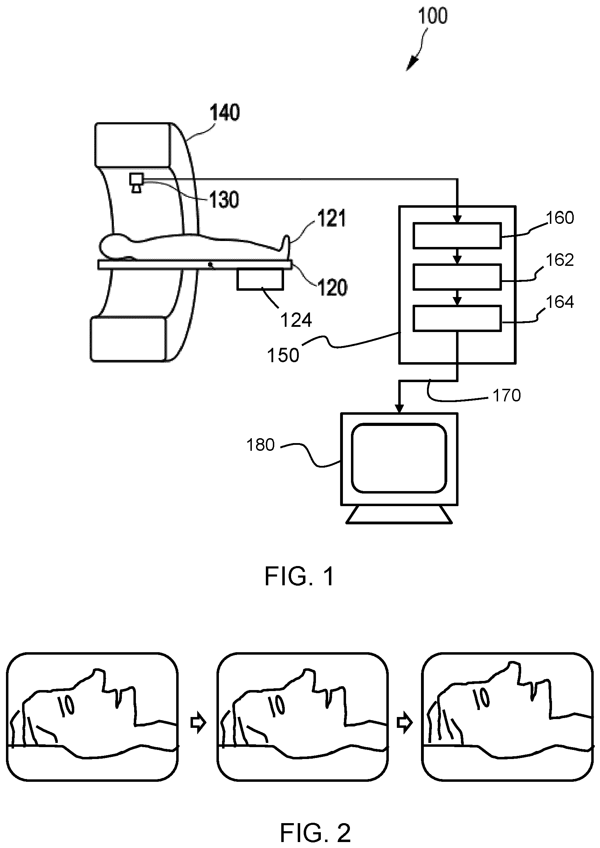

Fig. 1 shows schematically an example of a medical scanner 100 for acquiring a medical image of a subject and which additionally includes an imaging system for capturing optical images of the patient during the medical scan. The images are generated for the purposes of determining patient movement.

The medical scanner 100 comprises a scanning system such as a CT imaging system 140 that is adapted to acquire a medical image, i.e. a CT image in this example, of a patient 121 positioned on a patient support 120. The patient support 120 is adapted to move the patient 121 through the CT imaging system 140 during the CT imaging procedure. For this purpose, the medical scanner has a drive system 124 for driving the patient support through the scanning system.

The imaging system comprises an optical camera 130 that is adapted to acquire monitoring images of the patient 121 during the CT imaging procedure, and a processor 150. The operation of the system with a single camera will be explained, but there may be multiple cameras as explained further below. The camera can be a color or monochrome camera. It can use visible light or infrared light.

The camera 130 has a wide field of view so that it captures a full view of the patient support 120, or at least the part of the patient support 120 over which regions of interest of the patient will be positioned. Furthermore, the movement of the patient support 120 means that an even wider field of view is needed in order for the desired parts of the patient support to remain in the field of view of the camera (which is mounted statically to the scanning system) throughout the medical scan.

The camera for example comprises a fisheye lens with a field of view greater than 120 degrees, for example greater than 150 degrees, for example 160 degrees or more. Thus, the patient support (and the patient on the patient support) can be imaged either by a single camera as shown or by a small set of cameras.

The wide angle lens results in image distortion, such that the shape of objects appears differently at different regions of the field of view of the camera. As a result, the shape of objects or regions of interest will change when the position of those objects or regions of interest change in the field of view of the camera, as a result of the movement of the patient support.

To address this issue, the processor 150 performs image post-processing to correct those distortions within the captured images, i.e. resulting from the width of the field of view of the camera. This is shown as post-processing unit 160. For the purposes of the post-processing, the cameras are calibrated, such that geometric distortions are corrected by the post-processing, and the position and orientation of each camera with respect to the scanner coordinate system is known.

The processor tracks at least one region of interest of the patient during the patient support displacement. This is implemented by tracking unit 162. The (or each) region of interest is a body part to be monitored such as the face, an area to be scanned, an area of IV injection, or the hands. The location of the region of interest in an initial image can be defined manually or automatically by appropriate algorithms (e.g. using keypoint detection algorithms) which locate a selected anatomical feature of interest.

The processor then generates a distortion-corrected image sequence of the (or each) region of interest. This is implemented by an image sequence generator 164.

The movement of the patient support is used for the tracking and for generating the distortion-corrected image sequence. The motion of the patient support is for example provided by the scanning system.

The distortion-corrected image sequence is provided as output 170 for display on a display 180. This display 180 may be part of the medical scanner, or it may be a separate device such as a mobile device of a technician or it may be part of a remote operation center, to which the image data is transmitted by wired or wireless data transfer.

The output 170 comprises a continuous video stream. There may be a video stream of one or regions of interest.

Fig. 2 shows an example of three images of the distortion-corrected image sequence, in which the region of interest is the face of the patient. There may be separate image sequences for separate regions of interest.

As shown, the camera (or each camera) is oriented towards the patient support and covers a wide range of view. The position, orientation and intrinsic parameters of the cameras are obtained by a calibration step which is performed once at system installation. This calibration enables the required image post-processing to be derived, which in turn enables the region of interest to be tracked during patient support displacement.

The distortion-corrected image sequence is for example a zoomed-in crop of the overall distortion-corrected camera images and it offers a continuous view of the same anatomy.

EP 4295744 discloses an approach according to which, despite the movement of the patient support relative to the camera, the region of interest is located at a static location within the images that are cropped out of the distortion-corrected image sequence. The region of interest moves within the distortion-corrected images as a result of patient support displacement. After tracking the region of interest, a cropped image around the region of interest is generated, such that the body parts within this region of interest appear to be static. Thus, the cropped distortion-corrected image sequence appears to have been obtained from a camera which has travelled along with the patient support during the scan. Perspective correction is also discussed in EP 4295744 by tilting an output image in three dimensions.

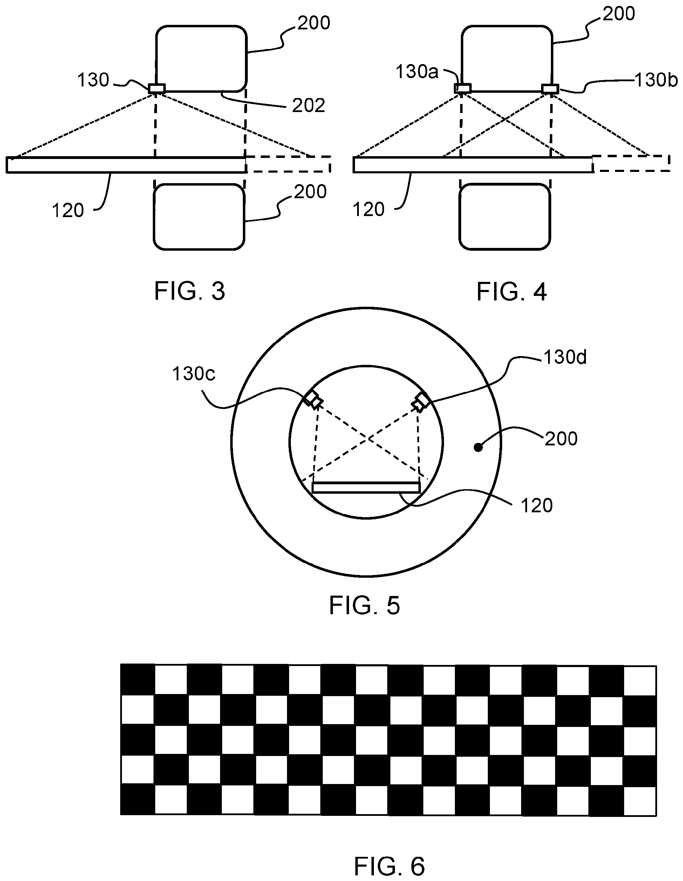

There are various options for camera placement. Fig. 3 shows a side view of a CT scanner with a single camera 130 located at one side of a gantry 200 having an opening 202 through which the patient support 120 extends.

The camera 130 has a field of view which is sufficiently wide in the length direction of the patent support to cover the whole length of the patient support, and the field of view is sufficiently wide in the width direction of the patient support to cover the whole width of the patient support. Furthermore, the field of view covers the whole patient support over its range of movement (thus the patient support 120 is represented in Fig. 3 by the full range of positions it may adopt).

The camera field of view may instead only cover regions of the patient support over which the regions of interest will be located, e.g. the face and abdomen, but again including all the positions of those regions of the patient support during displacement of the patient support.

Fig. 4 shows a side view of a medical scanner with two cameras; a first camera 130a located at one side of the gantry 200 and a second camera 130b located at an opposite side of the gantry, where the "sides" are at different positions along the patient support- i.e. one side faces the head end of the patient support and one side faces the foot end patient support.

There may also be multiple cameras even at a same position along the patient support. Fig. 5 shows two cameras 130c, 130d which may be located within the gantry bore, or located at one side of the gantry. They face the patient support but from a lateral side rather than from directly above. As explained further below, this ensures vertical motion is evident in the images. For example, the cameras may be oriented at 90 degrees relative to each other (in a plane perpendicular to the length axis of the patient support).

As explained above, a calibration process is used to derive the post-processing requirements to correct for image distortion. One approach is to apply a known pattern to the patient support, such as the checkerboard pattern shown in Fig. 6. Image distortions, particularly prominent at the edges of the field of view, may be corrected be deriving a correction function which returns the captured images to the known "correct" image. By calibrating the cameras with this checkerboard pattern, and combining the calibration result with the respective position of the patient support offered by the scanning system, the region of interest for one or more body parts can be tracked over time even when the patient support is moving, as explained above.

To the extent described above, the system is known, from EP 4295744. This invention provides improved image processing in the system described above. The invention may be implemented by the system of Fig. 1, with a different image processing implemented by the processor 150.

According to this invention there is again a set of one or more fixed optical cameras. A position input identifies a patient support position or provides patient position information relative to the imaging system.

Image post-processing is performed to create virtual views. The virtual views may be generated from desired positions and orientations and/or with a desired focal depth. **In** this way, when a virtual view image sequence is generated, depending on the patient support or patient position, the target view configuration may be adapted dynamically so as to offer the optimal virtual view to the region of interest. For example, a virtual camera yaw angle may be controlled in dependence on the patient support position. Also, the focal length may be increased when the target object is further away from the camera.

The virtual camera can be placed closer or further to the patient by varying the virtual camera position. It is also possible to zoom in or out to include more or less background (by varying the virtual focal length). The virtual camera can be rotated in any direction (pitch, roll, yaw angles) to change the perspective, to replicate a viewer turning their head. As in the system explained above, only a portion of the resulting image can be selected to crop around the ROI.

A virtual camera image is selected dynamically depending on the patient support position (rotating the perspective to follow the support motion). However, virtual views may also be selected statically (with no change during support motion) so that multiple views may be presented.

In this way, the imaging system digitally simulates the effect obtained by a motorized camera, making use of the large FOV of the static cameras. A virtual, but natural, traveling effect can be created, which appears as if the observer would turn the head to follow the moving object during patient support movement, such that the patient remains in the center of the image.

In-bore cameras are integrated inside the bore of the scanner, or at the front and rear sides of the gantry panel as described above. Each camera is equipped with a wide FOV lens (i.e. a fisheye lens). From each camera, virtual views each corresponding to a target view configuration are generated digitally. The target view configurations may be defined by camera geometry parameters such as pitch, yaw, roll, distance to the central object, virtual focal length, image size, pixel resolution.

As explained above with reference to Fig. 1, in-bore cameras are integrated inside the bore of the scanner, or at the front and rear sides of the gantry panel to acquire an unobstructed view of the patient for the different patient support positions. Each camera is equipped with a wide FOV lens, typically larger than 120 degrees. Fisheye lenses offer a 180 to 190 degree FOV.

From each camera, virtual views, each corresponding to a target view configuration, are generated digitally. Preferably, these virtual views are free of geometric distortions. Thus, in the manner explained above, the processor 150 is preferably configured to correct distortions within the captured images resulting at least from the width of the field of view of the camera or cameras. Each virtual view may select only a sub-part of the totally imaged FOV and be cropped around the region where the patient is best visible.

The target view configurations may be defined by at least one of the following camera geometry parameters: pitch, yaw, roll, distance to the central object, virtual focal length, image size, pixel resolution.

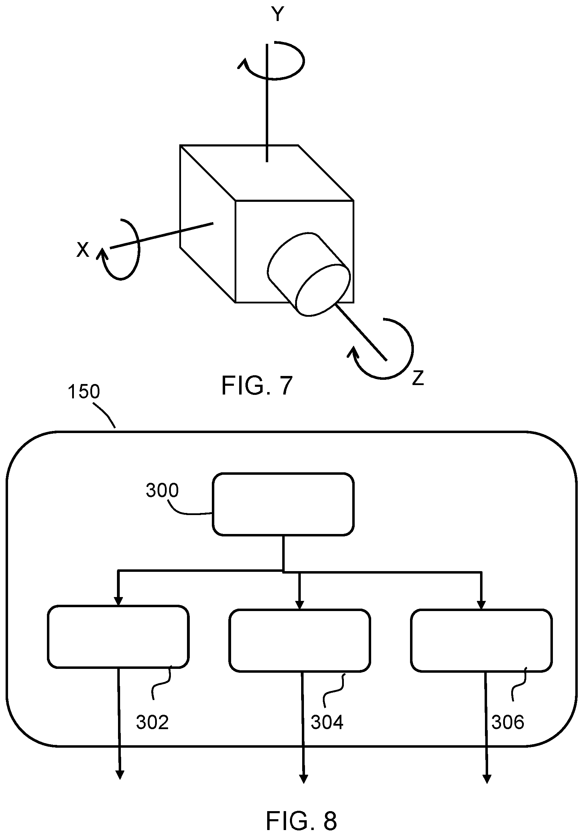

Fig. 7 shows pitch, roll and yaw parameters describing the orientation of a camera The pitch is shown as rotation about an x-axis, the yaw is rotation about a y-axis and the roll is rotation about a z-axis.

The pitch roll and yaw are defined as zero when the camera is horizontal and points to north. The pitch goes positive if the camera is pointing up and negative if the camera is pointing down. The yaw goes positive if the camera points east and negative if the camera points west. The roll is positive if the camera is rotated clockwise and negative if the camera is rotated anticlockwise.

The cameras are located in the bore with the optical axis of the camera (the z-axis) being perpendicular or close to perpendicular to the main axis of the bore cylinder, hence perpendicular to the patient support displacement direction.

The yaw angle is for example controlled in dependence on the patient support position (or more generally the patient position). The focal length may be increased when the target object is further away from the camera. The pitch may be adjusted to the vertical position of the patient support.

The camera can be anywhere on the bore diameter (at any angle, such as 12 o-clock, or 3 o-clock etc.,). One example makes use of two cameras at 1:30 and 10:30 positions (corresponding to +45 and -45 degrees from the top).

The virtual pitch of the camera (rotation about the x-axis) can be adjusted to look more downwards or upwards and therefore compensate for different patient support heights for example. The virtual yaw of the camera (rotation about the y-axis) can be adjusted to look more to the right (towards the front of the bore) or to the left (towards the rear). Thus, yaw selection can be used to follow the patient support as it moves in our out. The virtual roll of the cameras is for example used for correcting for inaccuracies in the positioning and orienting of the true camera hardware.

The computation of the virtual views is based on the one-time calibration of the camera parameters as explained above, for example by means of a chessboard pattern. Other patterns can be used as well.

In one implementation, the desired target view configuration is represented mathematically by a homography (a projective transformation between two planar projections of an image). Homographies are simple image transformations that describe the relative motion between two images, when the camera (or the observed object) moves. The homography parameters are computed based on the calibration procedure, and a re-mapping function from the original camera image to the target view image may be computed and preferably stored in memory.

Thus, one method to create such virtual views is to compute a homography transformation for each desired position, angulation and or focal depth.

The computation of the homography parameters can be done in the following manner:
an image with a predefined set of visible points is used as input, such as corners of a chessboard pattern.
an image processing method, like corner detection, is used to detect the set of points. This results in coordinates of the detected points.

Based on the desired position, angulation and focal depth of the virtual camera, the coordinates of the detected points in the desired virtual view are computed. Then, the homography transform is computed that maps the coordinates of the detected points to the coordinates of the same points in the virtual view.

The homography transformation cannot re-create image parts that are not visible in the original image, because they were obstructed. In that case, the missing image portion will be filled by the scene object that was causing the obstruction. However, even with such imperfection, the impression of a perspective change is created, and as long as the variations in the perspective remain close to the original perspective, an acceptable image output will result.

The application of the re-mapping function to the original camera image can be done for example by means of known re-map functions, such as the remap() function of OpenCV. This re-mapping step can be performed on CPU or on GPU.

Fig. 8 shows an example of the image processing performed by the processor 150 (instead of that shown in Fig. 1).

In step 300, an original camera image is obtained, such as a camera with a fisheye lens.

In step 302, image correction is carried out to correct for distortion resulting from the fisheye lens.

Steps 304, 306, 308 represent the generation of different possible virtual images by remapping of the distortion corrected image.

For example, images 304, 306, 308 may have different combinations of a radial distance D to the isocenter, focal length f, pitch angle and yaw angle.

The yaw angle is for example adapted dynamically in dependence on the position input, so as to simulate a rotation of the camera as the patient moves through the scanner bore.

As explained above, image processing is carried out to correct for distortions in the original image, for example resulting from the use of a fisheye lens. Distortion correction is desirable for human observers as well as for AI systems, which are normally trained on less distorted perspective images. Furthermore, the motion of the CT patient couch appears highly curved in the raw fisheye images. This not only creates an unnatural visual effect, but it also makes it more difficult to visually interpret and predict the motion of the couch.

A pin-hole camera model (perspective viewing) is defined by a fixed viewing plane and a focus, which is not in the plane: Each 3D point is mapped on the viewing plane by intersecting the line connecting the point and focus with the viewing plane. Points, which are in the plane through the focus and parallel to the viewing plane, cannot be mapped on the viewing plane. All other 3D points are mapped onto the viewing plane.

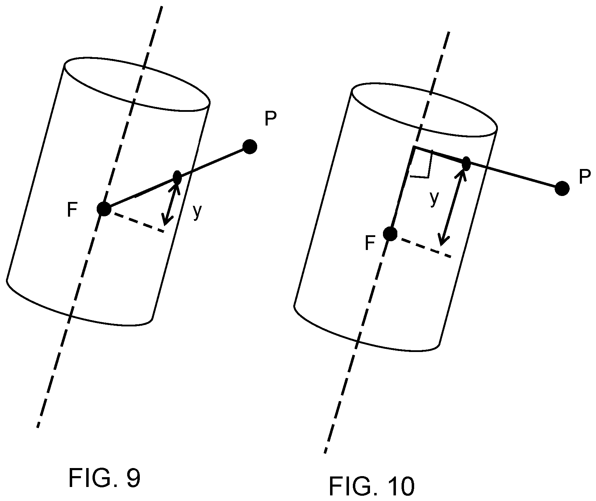

Fig. 9 shows a known projection of a 3D point P on a cylinder. In this cylindrical camera model, the planar viewing plane is replaced by a cylinder. Typically, the focus F is located on the cylinder axis. Except for the points on the cylinder axis, each 3D point is then mapped to an azimuthal angle [0°; 360°] and a polar angle.

Fig. 10 shows an alternative projection approach which will be described as a pseudo-cylindrical projection with the altitude (y-coordinate) defined by the orthogonal projection on the cylinder axis. In this way, distortions are corrected by performing a pseudo-cylindrical mapping comprising mapping from each point in space onto a cylinder surface but orthogonally towards the cylinder axis.

The z-axes of the cameras point toward the central line of the cylinder and are perpendicular to it. The x-axis of the cameras are parallel to the central line of the cylinder. The y-axes of the cameras are tangential to the bore inner circle. The exact orientation depends on the camera location.

The pseudo-cylindrical projection is applied after the fisheye lens distortion correction. It is an additional mapping, The pseudo-cylindrical mapping may be concatenated with the fish eye distortion correction in order to speed up the complete mapping.

For the deformation encountered, a point (x, y)T of the image plane I = [0; nx - 1] × [0; ny - 1] is mapped to the point (f(x), y) ε [0; Nx - 1] × [0; ny - 1].

The range of image pixels in the x-direction before the mapping is from 0 to nx-1, hence nx pixel in total, and is Nx-1 after the mapping. The range of image pixels in the y-direction is 0 to ny -1 before and after the mapping.

nx is the number of pixels in the x-direction in the input image. ny is the number of pixels in the input image and in the output image in y-direction. Nx is the number of pixels in x-direction in the output image.

f(x) is the function applied to the pixel only in the x-direction. This function is not dependent on the y-location. Thus, there is no deformation in the y-coordinates and horizontal lines remain horizontal.

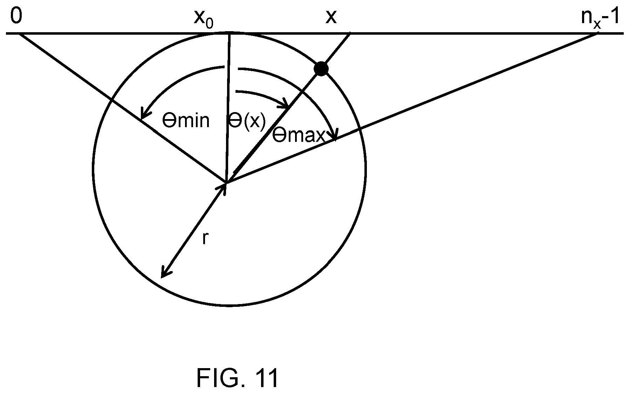

The mapping f(x) along the x-axis is illustrated in Fig. 11. It is a simple projection of the axis onto a circle at location x₀ with radius r. After the projection the range [θmin; θmax] is scaled to [0; Nx - 1]. The resulting map is defined by: $f \left(x\right) : = \frac{{N}_{x} - 1}{{θ}_{max} - {θ}_{min}} \left(θ\left(x\right)-{θ}_{min}\right)$ $θ \left(x\right) : = atan \frac{x - {x}_{0}}{r}$ ${θ}_{min} : = atan \frac{- {x}_{0}}{r}$ ${θ}_{max} : = atan \frac{{n}_{x} - 1 - {x}_{0}}{r}$

The size in pixels of the output image is chosen, so that there is no deformation at the cylinder axis, df(x₀)/dx = 1. ${N}_{x} = r ⋅ \left({θ}_{max}-{θ}_{min}\right) + 1$

In an original fisheye lens image, all originally straight lines appear curved so that visual interpretation as well as AI based recognition systems are not effective. A basic cylindrical projection means that straight lines are not straight anymore. The proposed pseudo-cylindrical projection results in both straight lines and realistic feature shapes.

The axis of the cylinder is oriented along the columns of the image. In a preferred embodiment, it is therefore perpendicular to the main axis of the CT bore. This has advantages because the main direction of interest for viewing, for which a larger FOV is preferably reconstructed, is the main axis of the CT bore.

The proposed pseudo-cylindrical transformation is defined by means of dedicated parameters, such as the radius of the cylinder, and the horizontal and vertical FOV. Different parameters can be chosen for different applications. For example, for a head scan where the patient support displacement is relatively small, the radius and FOV can be selected to generate a zoomed image around the region where the head with be confined. On the contrary, for CT applications with a large scan range, the radius and FOV can be selected so as to cover the overall expected patient support motion. Note that the corresponding mappings can be precomputed and stored on computer, so that the selection and application at run-time of a specific mapping does not require more computation time.

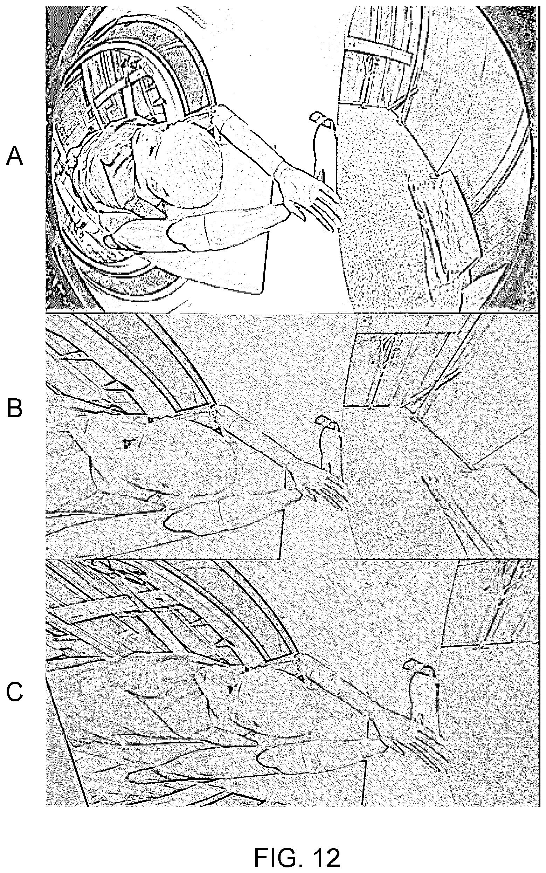

Fig. 12 shows an example of virtual view generation. Fig. 12A shows an original fisheye lens image. Fig. 12B shows a distortion-corrected virtual camera image with modified pitch and original yaw. Fig. 12C shows a distortion-corrected virtual camera image with modified pitch and modified yaw simulating a rotation of the camera to the left.

The invention may be applied to any medical scanner in which a patient support is moved during the imaging procedure, like a PET imaging device, an MR imaging device, a SPECT imaging device, as well as a CT scanner as described above. The medical scanner may comprise a C-arm or a closed bore. The camera or cameras may be located in the bore or at an inner surface of the C-arm, or outside the bore or C-arm envelope. However, in all cases, the camera is static relative to the main body of the scanning system and thus the patient support moves relative to the camera. The camera may not be mounted directly to the medical scanner but may instead be in a fixed position relative to the medical scanner by a separate mounting.

Although in the above described embodiments the patient support is always a patient support on which a patient is lying during the acquisition of the medical image, the patient support can also be configured for a sitting or a standing patient.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An imaging system for capturing images of a patient during a medical scan using a scanner having a scanning system (140) and a patient support (120) that is configured to move relative to the scanning system, the imaging system comprising:
a set of one or more optical cameras (130) for mounting in a fixed position relative to the scanning system directed towards the patient support for capturing images; and
a processor (150), **characterized in that** the processor is configured to:
receive a position input identifying a patient support position or providing patient position information relative to the imaging system;
perform image post-processing of the captured images to create virtual views, wherein the image post-processing comprises selecting (i) a position and orientation and/or (ii) a focal depth, for the virtual view generation in dependence on the position input;
generate at least one virtual view image sequence; and
output the virtual view image sequence for display.

2. The imaging system of claim 1, wherein the processor (150) is configured to correct distortions within the captured images resulting at least from the width of the field of view of the camera or cameras.

3. The imaging system of claim 2, wherein the processor (150) is configured to correct distortions by performing a pseudo-cylindrical mapping comprising mapping from each point in space onto a cylinder surface orthogonally towards the cylinder axis.

4. The imaging system of any one of claims 1 to 3, wherein the processor (150) is configured to create virtual views of a tracked region of interest of the patient.

5. The imaging system of claim 4, wherein the processor (150) is configured to perform the tracking using the position input.

6. The imaging system of claim 4 or 5, wherein the region or regions of interest comprise one or more of:
the face;
the hands;
the thorax, including the diaphragm region;
a body area to be scanned; and
a body area where there is an intravenous injection.

7. The imaging system of any one of claims 1 to 6, wherein the processor (150) is configured to adapt a virtual view orientation of the camera or cameras by adjusting one or more of a yaw angle, roll angle, pitch angle, camera position and focal length, for example by adjusting the yaw angle in dependence on the position input.

8. The imaging system of any one of claims 1 to 7, wherein at least one camera of the set comprises a fisheye lens with a field of view greater than 120 degrees, for example greater than 150 degrees.

9. The imaging system of any one of claims 1 to 8, wherein the processor (150) is configured to perform a calibration process which involves imaging one or more calibration images.

10. A medical scanner comprising:
a scanning system (140);
a patient support (120) extending through the scanning system;
a drive system (124) for driving the patient support through the scanning system; and
the imaging system of any one of claims 1 to 9.

11. The medical scanner of claim 10, wherein the set of cameras of the imaging system comprises:
a first camera mounted above the patient support at one side of the scanning system, facing towards the patient support; and optionally
a second camera mounted above the patient support at an opposite side of the scanning system, facing towards the patient support.

12. An imaging processing method for processing images of a patient during a medical scan using a scanner having a scanning system, a patient support and a set of one or more cameras mounted in a fixed position relative to the scanning system directed towards the patient support, **characterized in that** the method comprises:
receiving a position input identifying a patient support position or providing patient position information relative to the imaging system;
performing image post-processing of images captured by the set of one or more cameras to create virtual views, wherein the image post-processing comprises adapting (i) a position and orientation and/or (ii) a focal depth for the virtual view generation in dependence on the position input;
generating a virtual view image sequence; and
outputting the virtual view image sequence for display.

13. The method of claim 11, comprising correcting distortions within the captured images resulting at least from the width of the field of view of the camera or cameras and creating virtual views of a tracked region of interest of the patient.

14. The method of claim 13, comprising performing the tracking using the position input.

15. A computer program comprising computer program code which is configured, when the program is run on the processor of the imaging system of any one of claims 1 to 9, to implement the method of any one of claims 12 to 14.
